(19) Europäisches Patentamt
European Patent Office
Office européen des brevets

(11) Veröffentlichungsnummer: **0 000 050**
**A1**

(12) # EUROPÄISCHE PATENTANMELDUNG

(21) Anmeldenummer: **78100090.6**

(22) Anmeldetag: **05.06.78**

(51) Int. Cl.²: **C 07 C 79/46**, A 01 N 9/26, A 01 N 9/12, A 01 N 9/22, A 01 N 9/20, C 07 C 103/178, C 07 C 103/34

(30) Priorität: **07.06.77 DE 2725590**
**08.10.77 DE 2745462**

(43) Veröffentlichungstag der Anmeldung: **20.12.78 Patentblatt 78/1**

(84) Benannte Vertragsstaaten: **BE CH DE FR GB NL**

(71) Anmelder: **Bayer Aktiengesellschaft, Zentralbereich Patente, Marken und Lizenzen Bayerwerk, D-5090 Leverkusen 1 (DE)**

(72) Erfinder: **Förster, Heinz, Dr., Am Eckbusch 47, D-5600 Wuppertal 1 (DE)**

(72) Erfinder: **Eue, Ludwig, Dr., Paul-Klee-Strasse 36, D-5090 Leverkusen 1 (DE)**

(72) Erfinder: **Schmidt, Robert Rudolf, Dr., Hahnenweg 5, D-5000 Köln 80 (DE)**

(54) **4-(2-Chlor-4-nitrophenoxy)-phenoxyalkancarbonsäure-Derivate, Verfahren zu ihrer Herstellung und ihre Verwendung als Herbizide.**

(57) 4-(2-chlor-4-nitrophenoxy)- phenoxyalkancarbonsäure-Derivate der Formel

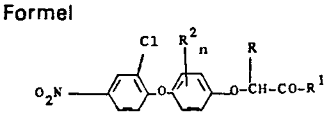

in welcher

R für Wasserstoff oder Alkyl,

R¹ für Alkoxy, das ein- oder mehrfach durch Hydroxyl, Halogen, Alkoxy, Alkoxy-alkoxy, Alkylthio, Amino, Monoalkylamino, Dialkylamino oder Carbonyl-N-alkyl- N-aryl substituiert ist, für Hydroxy-alkenoxy, Hydroxy-alkinoxy, Hydroxylamino, einen stickstoffhaltigen Heterocyclus, dessen Ring durch weitere Hetero-atome unterbrochen sein kann und/oder gegebenenfalls durch Alkyl oder Halogen substituiert sein kann,
Arylamino bzw. Monoalkyl-monoarylamino, in denen der Arylrest jeweils durch Alkyl, Nitro, Halogen oder Halogenalkyl substituiert sein kann,

R² für Wasserstoff, Halogen oder Alkyl und

n für eine ganze Zahl von 1 bis 4

stehen; Verfahren zu deren Herstellung und ihre Verwendung als Herbizide.

EP 0 000 050 A1

BAYER AKTIENGESELLSCHAFT 5090 Leverkusen, Bayerwerk
Zentralbereich DÜ-lz-AB
Patente, Marken und Lizenzen Typ Ib

# 4-(2-Chlor-4-nitrophenoxy)-phenoxyalkancarbonsäure-Derivate, Verfahren zu ihrer Herstellung und ihre Verwendung als Herbizide

Die vorliegende Erfindung betrifft neue 4-(2-Chlor-4-nitrophenoxy)-phenoxyalkancarbonsäure-Derivate, mehrere Verfahren zu ihrer Herstellung sowie ihre Verwendung als Herbizide.

Es ist bereits bekannt geworden, daß 4-(2,4-Dichlorphenoxy)-$\alpha$-phenoxypropionsäuremethylester zur Unkrautbekämpfung, vor allem bei grasartigen Unkräutern, verwendet werden kann (vergleiche Deutsche Offenlegungsschrift 2 223 894). Diese Verbindung ist jedoch, insbesondere bei niedrigen Aufwandmengen und -konzentrationen, nicht gegen alle Unkräuter ausreichend wirksam.

Ferner ist bereits bekannt geworden, daß bestimmte 4-(2-Chlor-4-nitrophenoxy)-phenoxyalkancarbonsäuren herbizide Eigenschaften besitzen (vgl. DT-OS 26 52 384). Die Wirksamkeit dieser Stoffe ist jedoch ebenfalls nicht immer befriedigend.

Es wurden nun die neuen 4-(2-Chlor-4-nitrophenoxy)-phenoxy-alkancarbonsäure-Derivate der Formel

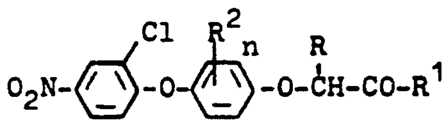

(I)

gefunden,

in welcher

R für Wasserstoff oder Alkyl,

$R^1$ für Alkoxy, das ein- oder mehrfach durch Hydroxyl, Halogen, Alkoxy, Alkoxy-alkoxy, Alkylthio, Amino, Monoalkylamino, Dialkylamino oder Carbonyl-N-alkyl-N-aryl substituiert ist, für Hydroxy—alkenoxy, Hydroxy-alkinoxy, Hydroxylamino, einen stickstoffhaltigen Heterocyclus, dessen Ring durch weitere Heteroatome unterbrochen sein kann und/oder gegebenenfalls durch Alkyl oder Halogen substituiert sein kann, Arylamino bzw. Monoalkyl-monoarylamino, in denen der Arylrest jeweils durch Alkyl, Nitro, Halogen oder Halogenalkyl substituiert sein kann,

$R^2$ für Wasserstoff, Halogen oder Alkyl und

n für eine ganze Zahl von 1 bis 4

stehen.

Diese Verbindungen zeichnen sich durch starke herbizide Eigenschaften aus.

Weiterhin wurde gefunden, daß die 4-(2-Chlor-4-nitrophenoxy)-phenoxyalkancarbonsäure-Derivate (I) erhalten werden, wenn man

a) 4-(2-Chlor-4-nitrophenoxy)-phenoxyalkancarbonsäurehalogenide der Formel (II)

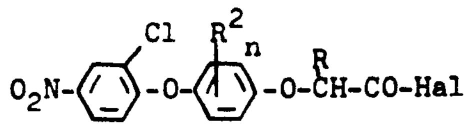

(II)

in welcher

$R$, $R^2$ und $n$ die oben angegebene Bedeutung haben und
Hal für Halogen, vorzugsweise Chlor, steht,

mit Verbindungen der Formel (III)

$$HR^1 \qquad (III)$$

in welcher

$R^1$ die oben angegebene Bedeutung hat,

gegebenenfalls in Gegenwart eines Säureakzeptors oder gegebenenfalls in Form der Alkali-, Erdalkali- oder Ammoniumsalze und gegebenenfalls in Gegenwart eines Lösungs- oder Verdünnungsmittels umsetzt, oder

b) 2-Chlor-4-nitro-4'-hydroxy-diphenyläther der Formel (IV)

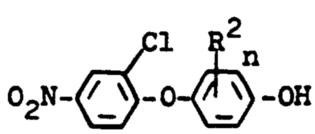

(IV)

in welcher

$R^2$ und $n$ die oben angegebene Bedeutung haben, gegebenenfalls in Form der Alkali- bzw. Erdalkalisalze mit $\alpha$-Halogenalkancarbonsäuren oder Derivaten der Formel (V)

$$Hal^1-\overset{R}{\overset{|}{C}}H-CO-R^1 \qquad (V)$$

in welcher

R und $R^1$ die oben angegebene Bedeutung haben und

$Hal^1$ für Halogen, vorzugsweise Chlor oder Brom,

steht, gegebenenfalls in Gegenwart eines Säureakzeptors und gegebenenfalls in Gegenwart eines Lösungs- oder Verdünnungsmittels umsetzt oder

c) 4-(2-Chlor-4-nitrophenoxy)-phenoxyalkancarbonsäure-methylester der FormelVI

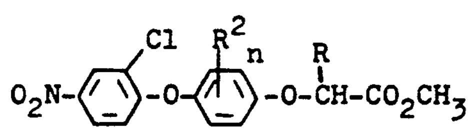

(VI)

in welcher

R, $R^2$ und n die oben angegebene Bedeutung haben,

mit Alkoholen der Formel VII

$$HOR^3 \qquad (VII)$$

in welcher

$R^3$ für substituiertes Alkyl mit mehr als einem Kohlenstoff steht,

gegebenenfalls in Gegenwart eines Alkoholats und gegebenenfalls in Gegenwart eines Lösungs- oder Verdünnungsmittels umsetzt, wobei die zwischenzeitliche Isolierung des Methylesters nicht unbedingt erforderlich ist, sondern die Umsetzung eines 2-Chlor-4-nitro-4'-hydroxy-diphenyläthers (IV) mit dem α-Halogenalkancarbonsäuremethylester und die anschließende Umesterung als Eintopfverfahren durchgeführt werden kann oder

d) die Verbindungen der Formel (VI) mit Hydroxylamin-Hydrohalogenid der Formel (VIII)

$$H_2NOH \times HHalogen \qquad (VIII)$$

gegebenenfalls in Gegenwart eines Säureakzeptors und gegebenenfalls in Gegenwart eines Lösungs- oder Verdünnungsmittels umsetzt.

Überraschenderweise besitzen die erfindungsgemäßen 4-(2-Chlor-4-nitrophenoxy)-phenoxyalkancarbonsäure-Derivate eine bessere herbizide Wirkung gegen Gräser und eine höhere Selektivität bei einer Reihe wichtiger Kulturpflanzen wie Getreide, Mais und Rüben, als die aus der Literatur vorbekannten Verbindungen analoger Konstitution und gleicher Wirkungsrichtung. Die erfindungsgemäßen Stoffe stellen somit eine wesentliche Bereicherung der Technik dar.

Verwendet man beispielsweise nach Verfahrensvariante a) 2,6-Dichlor-4-(2-chlor-4-nitrophenoxy)-α-phenoxypropionsäurechlorid und 2-Methyl-piperidin, nach Verfahrensvariante b) 2-Chlor-4-nitro-3'-chlor-4'-hydroxy-diphenyläther und

α-Brom-propionsäure-N-methyl-anilid, nach Verfahrensvariante c) 4-(2-Chlor-4-nitrophenoxy)-α-phenoxy-propionsäure-methylester und 2-Chloräthanol bzw. nach Verfahrensvariante d) 4-(2-Chlor-4-nitrophenoxy)-α-phenoxy-propionsäure-methylester und Hydroxylamin-Hydrochlorid als Ausgangsstoffe, so kann der Reaktionsverlauf durch die folgenden Formelschemata wiedergegeben werden:

a)
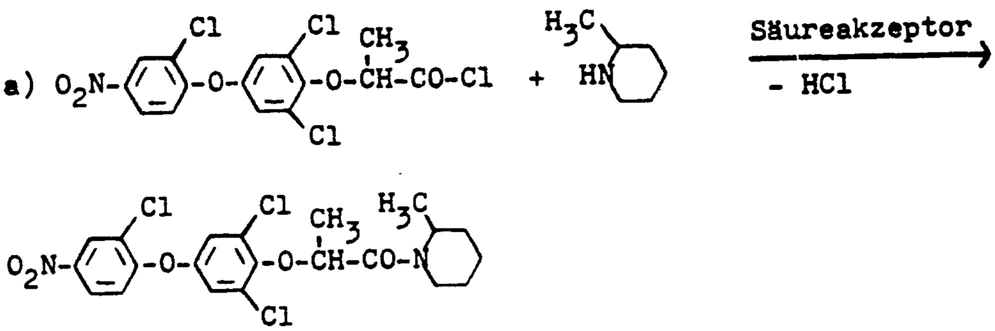


b)
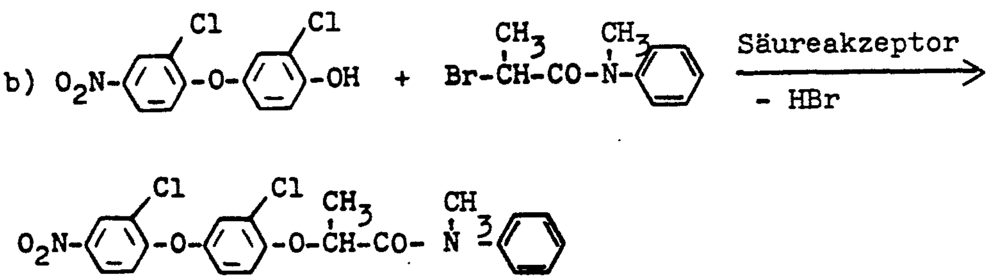


c)
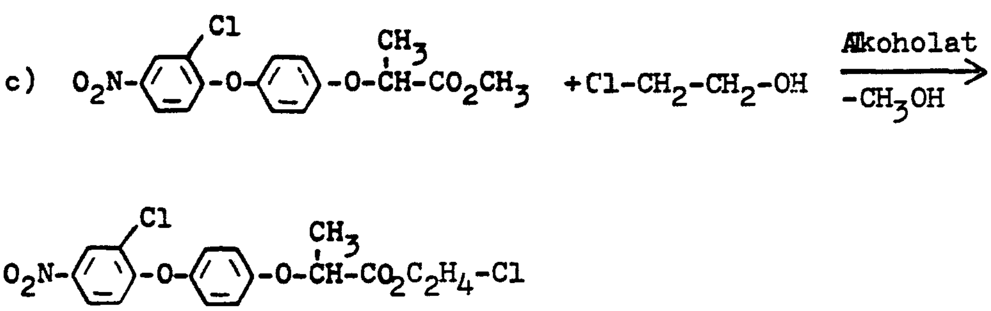

d) 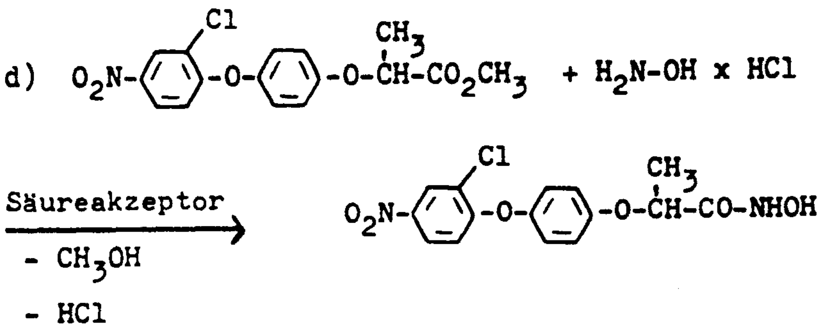


Die zu verwendenden Ausgangsstoffe sind durch die Formeln (II) bis (VIII) allgemein definiert. Vorzugsweise stehen darin jedoch

R für geradkettiges oder verzweigtes Alkyl mit 1 bis 8, insbesondere 1 bis 4, Kohlenstoffatomen,

$R^1$ für Hydroxylamino, geradkettiges oder verzweigtes Hydroxylalkoxy mit 1 bis 6, insbesondere 1 bis 4, Kohlenstoffatomen, geradkettiges oder verzweigtes Halogenalkoxy mit 1 bis 6, insbesondere 1 bis 4, Kohlenstoffatomen und 1 bis 5 Halogenatomen, insbesondere Chlor- oder Bromatomen, weiterhin für geradkettiges oder verzweigtes Alkoxyalkoxy und 1 bis 6, insbesondere 1 bis 4, Kohlenstoffatomen in jeder Alkoxygruppe, Dialkoxy-alkoxy mit 1 bis 6, insbesondere 1 bis 4, Kohlenstoffatomen in jeder Alkoxygruppe, geradkettiges oder verzweigtes (Alkoxyalkoxy)-alkoxy mit 1 bis 6, insbesondere 1 bis 4, Kohlenstoffatomen in jeder Alkoxygruppe, geradkettiges oder verzweigtes Alkylthioalkoxy mit jeweils 1 bis 6, insbesondere 1 bis 4, Kohlenstoffatomen in der Alkoxy- bzw. Alkylthio-Gruppe, geradkettiges oder verzweigtes Aminoalkoxy mit 1 bis 6, insbesondere 1 bis 4, Kohlenstoffatomen, ferner für Mono- oder Dialkylamino-alkoxy mit 1 bis 6, insbesondere 1 bis 4, Kohlenstoffatomen je Alkyl- bzw. Alkoxyrest, (Carbonyl-N-alkyl-N-phenyl)-alkoxy mit 1 bis 6, insbesondere 1 bis 4, Kohlenstoffatomen in der Alkyl- bzw. Alkoxygruppe, weiterhin für geradkettiges oder verzweigtes Hydroxy-alkenoxy mit 2 bis 6, insbesondere 2 bis 4, Kohlenstoffatomen, geradkettiges oder verzweigtes Hydroxy-alkinoxy mit 2 bis 6, insbesondere 2 bis 4, Kohlenstoffatomen, außerdem für einen Piperidinrest, 2-Methylpiperidinrest, Morpholinrest, Thiazinrest, Pyrazolrest, bzw. Monophenylaminorest, worin der Phenylrest gegebenenfalls ein- oder mehrfach, gleich oder verschieden durch Nitro, Chlor, Fluor, geradkettiges oder verzweigtes Alkyl bzw. Halogenalkyl mit 1 bis 6, insbesondere 1 bis 3, Kohlenstoffatomen substituiert sein kann, sowie für Monoalkyl-monophenylamino, wobei der Phenylrest wie oben substituiert sein kann und der Alkylrest 1 bis 6, insbesondere 1 bis 3, Kohlenstoffatome enthält,

$R^2$ für Wasserstoff, Chlor, Brom, Fluor, Methyl oder Äthyl,

$R^3$ für geradkettiges oder verzweigtes Alkyl mit 2 bis 10 Kohlenstoffatomen, das durch Chlor, Brom, Hydroxy, Alkoxy mit 1 bis 4 C-Atomen und/oder Alkylthio mit 1 bis 4 C-Atomen substituiert ist, und

n für eine ganze Zahl von 1 bis 4.

Besonders bevorzugt sind jedoch Verbindungen, in denen R für Wasserstoff oder Methyl, $R^1$ für Alkoxy mit 1-6 C-Atomen, das durch Chlor, Brom, Hydroxy, Alkoxy mit 1 bis 4 C-Atomen und/oder Alkylthio mit 1 bis 4 C-Atomen substituiert ist, und $R^2$ für Wasserstoff steht.

Die als Ausgangsmaterialien zu verwendenden 4-(2-Chlor-4-nitrophenoxy)-phenoxyalkancarbonsäurehalogenide (II) können nach Analogieverfahren aus den freien Säuren durch Umsetzen mit einem Halogenierungsmittel, wie Thionylchlorid oder Chlor, gewonnen werden. Als Beispiele dafür seien im einzelnen genannt:

4-(2-Chlor-4-nitrophenoxy)-phenoxy-essigsäurechlorid,
4-(2-Chlor-4-nitrophenoxy)-α-phenoxy-propionsäurechlorid,
4-(2-Chlor-4-nitrophenoxy)-α-phenoxy-n-buttersäurechlorid,
4-(2-Chlor-4-nitrophenoxy)-α-phenoxy-iso-buttersäurechlorid,
4-(2-Chlor-4-nitrophenoxy)-α-phenoxy-n-valerian-säurechlorid,
4-(2-Chlor-4-nitrophenxoy)-α-phenoxy-iso-valeriansäurechlorid,
4-(2-Chlor-4-nitrophenoxy)-(2-chlorphenoxy)-essigsäurechlorid,
4-(2-Chlor-4-nitrophenoxy)-α-(2-chlorphenoxy)-propionsäurechlorid,
4-(2-Chlor-4-nitrophenoxy)-α-(2-chlorphenoxy)-n-buttersäurechlorid,
4-(2-Chlor-4-nitrophenoxy)-α-(2-chlorphenoxy)-iso-buttersäurechlorid,
4-(2-Chlor-4-nitrophenoxy)-α-(2-chlorphenoxy)-n-valeriansäurechlorid,
4-(2-Chlor-4-nitrophenoxy)-α-(2-chlorphenoxy)-iso-valeriansäurechlorid,
4-(2-Chlor-4-nitrophenoxy)-(2,6-dichlorphenoxy)-essigsäurechlorid,
4-(2-Chlor-4-nitrophenoxy)-α-(2,6-dichlorphenoxy)-propionsäurechlorid,
4-(2-Chlor-4-nitrophenoxy)-α-(2,6-dichlorphenoxy)-n-buttersäurechlorid,
4-(2-Chlor-4-nitrophenoxy)-α-(2,6-dichlorphenoxy)-iso-buttersäurechlorid,
4-(2-Chlor-4-nitrophenoxy)-α-(2,6-dichlorphenoxy)-n-valeriansäurechlorid,
4-(2-Chlor-4-nitrophenoxy)-α-(2,6-dichlorphenoxy)-iso-valeriansäurechlorid,
4-(2-Chlor-4-nitrophenoxy)-(2-methylphenoxy)-essigsäurechlorid,
4-(2-Chlor-4-nitrophenoxy)-α-(2-methylphenoxy)-propionsäurechlorid,
4-(2-Chlor-4-nitrophenoxy)-α-(2-methylphenoxy)-n-buttersäurechlorid,
4-(2-Chlor-4-nitrophenoxy)-α-(2-methylphenoxy)-iso-buttersäurechlorid,
4-(2-Chlor-4-nitrophenoxy)-α-(2-methylphenoxy)-n-valeriansäurechlorid,

4-(2-Chlor-4-nitrophenoxy)-α-(2-methylphenoxy)-iso-valeriansäurechlorid,
4-(2-Chlor-4-nitrophenoxy)-(2-äthylphenoxy)-essigsäurechlorid,
4-(2-Chlor-4-nitrophenoxy)-α-(2-äthylphenoxy)-propionsäurechlorid,
4-(2-Chlor-4-nitrophenoxy)-α-(2-äthylphenoxy)-n-buttersäurechlorid,
4-(2-Chlor-4-nitrophenoxy)-α-(2-äthylphenoxy)-iso-buttersäurechlorid,
4-(2-Chlor-4-nitrophenoxy)-α-(2-äthylphenoxy)-n-valeriansäurechlorid,
4-(2-Chlor-4-nitrophenoxy)-α-(2-äthylphenoxy)-iso-valeriansäurechlorid,
4-(2-Chlor-4-nitrophenoxy)-(2,6-dimethylphenoxy)-essigsäurechlorid,
4-(2-Chlor-4-nitrophenoxy)-α-(2,6-dimethylphenoxy)-propionsäurechlorid,
4-(2-Chlor-4-nitrophenoxy)-α-(2,6-dimethylphenoxy)-n-buttersäurechlorid,
4-(2-Chlor-4-nitrophenoxy)-α-(2,6-dimethylphenoxy)-iso-buttersäurechlorid,
4-(2-Chlor-4-nitrophenoxy)-α-(2,6-dimethylphenoxy)-n-valeriansäurechlorid,
4-(2-Chlor-4-nitrophenoxy)-α-(2,6-dimethylphenoxy)-iso-valeriansäurechlorid,
4-(2-Chlor-4-nitrophenoxy)-(2,6-diäthylphenoxy)-essigsäurechlorid,
4-(2-Chlor-4-nitrophenoxy)-α-(2,6-diäthylphenoxy)-propionsäurechlorid,
4-(2-Chlor-4-nitrophenoxy)-α-(2,6-diäthylphenoxy)-n-buttersäurechlorid,
4-(2-Chlor-4-nitrophenoxy)-α-(2,6-diäthylphenoxy)-iso-buttersäurechlorid,

4-(2-Chlor-4-nitrophenoxy)-$\alpha$-(2,6-diäthylphenoxy)-n-valeriansäurechlorid und
4-(2-Chlor-4-nitrophenoxy)-$\alpha$-(2,6-diäthylphenoxy)-iso-valeriansäurechlorid.

Die weiterhin als Ausgangsverbindungen zu verwendenden Verbindungen (III) sind bekannt und auch im technischen Maßstab gut herstellbar. Als Beispiele dafür seien im einzelnen genannt:

Anilin, 2-Chlor-anilin, 3-Chlor-anilin, 4-Chlor-anilin, 2,3-Dichlor-anilin, 2,4-Dichlor-anilin, 3,4-Dichlor-anilin, 3,5-Dichlor-anilin, 2,4,5-Trichlor-anilin, 2-Nitro-anilin, 3-Nitro-anilin, 4-Nitro-anilin, 2-Nitro-4-chlor-anilin, 3-Nitro-4-chlor-anilin, 2-Chlor-4-nitro-anilin, 2,4-Dichlor-6-nitro-anilin, 2-Methyl-anilin, 2-Chlor-6-methyl-anilin, 3-Chlor-6-methyl-anilin, 3,4-Dichlor-6-methyl-anilin, 2-Methyl-6-nitro-anilin, 2-Methyl-4-nitro-anilin, 2-Methyl-3-nitro-anilin, 3-Chlor-6-methyl-4-nitro-anilin, 3-Methyl-anilin, 4-Methylanilin, 2,3-Dimethyl-anilin, 3,4-Dimethyl-anilin, 2,6-Dimethyl-anilin, 2,4-Dimethyl-anilin, 2,4,6-Trimethyl-anilin, 2,6-Diäthyl-anilin, 2,6-Diäthyl-4-methyl-anilin, Hydroxylamin, N-Methylanilin, N-Äthyl-anilin, N-n-Propyl-anilin, N-n-Butyl-anilin, N-iso-Butyl-anilin, N-Äthyl-2-chlor-anilin, N-Methyl-3-nitro-anilin, N-Methyl-4-nitro-anilin, N-Äthyl-4-nitro-anilin, N-Methyl-2-methyl-anilin, N-Äthyl-2-methyl-anilin, N-Methyl-2-methyl-5-nitro-anilin, N-Äthyl-2-methyl-5-nitro-anilin, N-Äthyl-3-methyl-anilin, N-Methyl-4-methyl-anilin, N-Äthyl-4-methyl-anilin, Äthandiol(1,2), 2-Chlor-äthanol, 2,2,2-Trichloräthanol, 1,3-Dichlor-propanol(2), 2-Methylamino-äthanol, 2-Dimethylamino-äthanol, 2-Diäthylamino-äthanol, 1-Dimethylamino-propanol(2), Piperidin,

2-Methylpiperidin, Morpholin, Thiazin, Pyrazol.

Weiterhin werden die 2-Chlor-4-nitro-4'-hydroxy-diphenyläther (IV) als Ausgangsverbindungen verwendet, die aus den 1,2-Dichlor-4-nitrophenyl und 1,4-Dihydroxyphenyl und Folgereaktionen, wie Halogenierung, hergestellt werden können. Als Beispiele seien im einzelnen genannt:
2-Chlor-4-nitro-4'-hydroxy-diphenyläther,
2-Chlor-4-nitro-3'-chlor-4'-hydroxy-diphenyläther,
2-Chlor-4-nitro-3',5'-dichlor-4'-hydroxy-diphenyläther,
2-Chlor-4-nitro-3'-methyl-4'-hydroxy-diphenyläther,
2-Chlor-4-nitro-3'-äthyl-4'-hydroxy-diphenyläther,
2-Chlor-4-nitro-3',5'-dimethyl-4'-hydroxy-diphenyläther,
2-Chlor-4-nitro-3',5'-diäthyl-4'-hydroxy-diphenyläther.

Weiterhin werden die bekannten $\alpha$-Halogenalkancarbonsäure-Derivate (V) als Ausgangsverbindungen verwendet. Als Beispiele dafür seien im einzelnen genannt:

2-Chlorpropionsäure-N-methyl-anilid und 2-Brompropionsäure-N-methyl-anilid.

Die 4-(2-Chlor-4-nitrophenoxy)-phenoxyalkancarbonsäuremethylesterderivate (VI) können nach Analogieverfahren hergestellt werden. Als Beispiele dafür seien im einzelnen genannt:
4-(2-Chlor-4-nitrophenoxy)-phenoxy-essigsäuremethylester,
4-(2-Chlor-4-nitrophenoxy)-$\alpha$-phenoxy-propionsäuremethylester,
4-(2-Chlor-4-nitrophenoxy)-$\alpha$-phenoxy-buttersäuremethylester,
4-(2-Chlor-4-nitrophenoxy)-(2-chlorphenoxy)-essigsäuremethylester,

4-(2-Chlor-4-nitrophenoxy)-α-(2-chlorphenoxy)-propionsäuremethylester,
4-(2-Chlor-4-nitrophenoxy)-α-(2-chlorphenoxy)-buttersäuremethylester,
4-(2-Chlor-4-nitrophenoxy)-(2,6-dichlorphenoxy)-essigsäuremethylester,
4-(2-Chlor-4-nitrophenoxy)-α-(2,6-dichlorphenoxy)-propionsäuremethylester,
4-(2-Chlor-4-nitrophenoxy)-α-(2,6-dichlorphenoxy)-buttersäuremethylester.

Das weiterhin zu verwendende Hydroxylamin (VIII) ist bekannt, ebenso wie die Alkohole (VII). Für letztere seien im einzelnen genannt:
2-Chloräthanol, 2,2-Dichloräthanol und 2,2,2-Trichloräthanol.

Die erfindungsgemäße Verfahrensvariante a) zur Herstellung der neuen 4-(2-Chlor-4-nitrophenoxy)-phenoxyalkancarbonsäure-Derivate wird bevorzugt unter Mitverwendung geeigneter Lösungs- oder Verdünnungsmittel durchgeführt. Als solche kommen praktisch alle inerten organischen Solventien infrage. Hierzu gehören insbesondere aliphatische oder aromatische Kohlenwasserstoffe, wie Petroläther, Benzin, Benzol, Toluol und Xylol, ferner chlorierte aliphatische oder aromatische Kohlenwasserstoffe, wie Methylenchlorid, Chloroform, Tetrachlorkohlenstoff, Äthylenchlorid und Chlorbenzol, weiterhin Äther, wie Diäthyläther, Dibutyläther, Tetrahydrofuran, Dioxan, darüber hinaus Ketone, wie Aceton, Methyläthylketon, Methyl-isopropylketon und Methyl-isobutylketon, außerdem Nitrile, wie Aceto- und Propionitril, und manchmal auch Alkohole, wie Methanol, Äthanol, n- oder iso-Propanol und Butanol.

Bei der Verfahrensvariante b) können grundsätzlich die

unter a) aufgeführten Lösungs- oder Verdünnungsmittel verwendet werden, außerdem z.B. Dimethylsulfoxid. Bei der Verfahrensvariante c) können die als Reaktionskomponente (VII) zu verwendenden Alkohole im Überschuß verwendet werden. Verfahrensvariante d) wird vorzugsweise in Gegenwart eines Alkohols, wie Methanol, durchgeführt.

Als Säureakzeptoren können bei der Umsetzung der Verfahrensvariante a) alle üblichen Säurebindemittel Verwendung finden. Besonders bewährt haben sich Alkalicarbonate oder -alkoholate, wie Kalium- oder Natriumcarbonat, Natrium- oder Kaliummethylat, Natrium- oder Kaliumäthylat, Kalium-tert.-butylat, ferner aliphatische, aromatische oder heterocyclische Amine, wie Triäthylamin, Trimethylamin, Dimethylanilin, Dimethylbenzylamin und Pyridin. Ferner kann in den entsprechenden Fällen auch ein Überschuß des gleichzeitig als Reaktionskomponente eingesetzten Amins, wie Piperidin, als Säureakzeptor dienen. Bei der Verfahrensvariante b) können alle üblichen Säurebindemittel Verwendung finden. Besonders bewährt haben sich Alkalicarbonate oder -alkoholate, wie Kalium- oder Natriumcarbonat, Natrium- oder Kaliummethylat, Natrium- oder Kaliumäthylat, Kalium-tert.-butylat, ferner aliphatische, aromatische oder heterocyclische Amine, wie Triäthylamin, Trimethylamin, Dimethylanilin, Dimethylbenzylamin und Pyridin, und bei der Umesterungsreaktion c) arbeitet man im allgemeinen in Gegenwart eines Alkoholates, wie Natriummethylat.

Die Reaktionstemperaturen können innerhalb eines größeren Bereichs variiert werden. Im allgemeinen arbeitet man bei 0 bis 150°C. Bei Verfahrensvariante a) vorzugsweise zwischen 0 und 50°C, bei Verfahrensvariante b) zwischen 80 und 110°C, bei Verfahrensvariante c) zwischen 60 und 120°C, und bei Ver-

fahrensvariante d) zwischen o und 2o°C.

Die erfindungsgemäßen Umsetzungen werden im allgemeinen bei Normaldruck durchgeführt.

Bei der Durchführung der erfindungsgemäßen Verfahrensvarianten a), b), c) bzw. d) setzt man die Reaktionskomponenten (III), (V), (VII) bzw. (VIII) im allgemeinen im Überschuß ein. Bei der Verfahrensvariante a) legt man vorzugsweise die Phenoxyalkancarbonsäurehalogenidkomponente (II) in einem der angegebenen Lösungsmittel gegebenenfalls in Gegenwart eines Säureakzeptors vor und tropft die Komponente (III) zu, handelt es sich bei der Komponente (III) um ein Amin, so wird vorzugsweise die Aminlösung vorgelegt. Im allgemeinen wird die Reaktionsmischung bei den angegebenen Temperaturen ein bis mehrere Stunden nachgerührt und dann erfolgt die Aufarbeitung wie üblich. Im allgemeinen wird die Reaktionsmischung direkt oder nach dem Einengen im Vakuum mit einem organischen Lösungsmittel, wie z.B. Toluol, versetzt, die organische Phase wird gewaschen und im Vakuum das Lösungsmittel abdestilliert. Bei der Verfahrensvariante b) wird nach mehrstündigem Rühren der Reaktionsmischung bei erhöhten Temperaturen, die Reaktionsmischung in Wasser gegossen und der ausfallende Niederschlag abgesaugt.

Bei der Verfahrensvariante c) wird die Reaktionsmischung nach beendeter Reaktion eingeengt, mit Wasser verrührt und abdekantiert. Mit Aceton verrührt, filtriert und eingeengt. Der Rückstand wird in einem organischen Lösungsmittel, wie z.B. Methylenchlorid, aufgenommen und die organische Phase wie üblich durch Waschen, Trocknen und Abdestillieren des Lösungsmittels aufgearbeitet.

Bei der Verfahrensvariante d) wird der Phenoxyalkancarbonsäuremethylester in Methanol vorgelegt, das Hydroxylamin x Hydrohalogenid in Wasser zugetropft und anschließend wird eine wässrige Natriumhydroxydlösung zugegeben. Dann gießt man in Wasser, stellt auf pH 5 ein und saugt den Niederschlag ab.

Einige Verbindungen fallen in kristalliner Form an und werden durch ihren Schmelzpunkt charakterisiert. Einige Verbindungen fallen in Form von Ölen an, die sich meistens nicht unzersetzt destillieren lassen. In diesem Fall kann man die Reinigung so durchführen, daß man die Rohprodukte durch sogenanntes "Andestillieren", d.h. durch längeres Erhitzen unter vermindertem Druck auf mäßig erhöhte Temperaturen von den letzten flüchtigen Anteilen befreit. Zur Charakterisierung der öligen Produkte dient der Brechungsindex.

Die erfindungsgemäßen Wirkstoffe beeinflussen das Pflanzenwachstum und können deshalb als Defoliants, Desiccants, Krautabtötungsmittel, Keimhemmungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind.

Die erfindungsgemäßen Wirkstoffe können z.B. bei den folgenden Pflanzen verwendet werden:

Dikotyle Unkräuter der Gattungen: Senf (Sinapis), Kresse (Lepidium), Labkraut (Galium), Sternmiere (Stellaria), Kamille (Matricaria), Hundskamille (Anthemis), Knopfkraut (Galinsoga), Gänsefuß (Chenopodium), Brennessel (Urtica), Kreuzkraut (Senecio), Fuchsschwanz (Amaranthus), Portulak (Portulaca), Spitzklette (Xanthium), Winde (Convolvulus), Prunkwinde (Ipomoea), Knöterich (Polygonum), Sesbanie (Sesbania), Ambrosie (Ambrosia), Kratzdistel (Cirsium), Distel (Carduus), Gänsedistel (Sonchus), Nachtschatten (Solanum), Sumpfkresse (Rorippa), Rotala, Büchsenkraut (Lindernia), Taubnessel (Lamium), Ehrenpreis (Veronica), Schönmalve (Abutilon), Emex, Stechapfel (Datura), Veilchen (Viola), Hanfnessel, Hohlzahn (Galeopsis), Mohn (Papaver), Flockenblume (Centaurea).

Dicotyle Kulturen der Gattungen: Baumwolle (Gossypium), Sojabohne (Glycine), Rübe (Beta), Möhre (Daucus), Gartenbohne (Phaseolus), Erbse (Pisum), Kartoffel (Solanum), Lein (Linum), Prunkwinde (Ipomoea), Bohne (Vicia), Tabak (Nicotiana), Tomate (Lycopersicon), Erdnuß (Arachis), Kohl (Brassica), Lattich (Lactuca), Gurke (Cucumis), Kürbis (Cuburbita).

Monokotyle Unkräuter der Gattungen: Hühnerhirse (Echinochloa), Borstenhirse (Setaria), Hirse (Panicum), Fingerhirse (Digitaria), Lieschgras (Phleum), Rispengras (Poa), Schwingel (Festuca), Eleusine, Brachiaria, Lolch (Lolium), Trespe (Bromus), Hafer (Avena), Zypergras (Cyperus), Mohrenhirse (Sorghum), Quecke (Agropyron), Hundszahngras (Cynodon), Monocharia, Fimbristylis, Pfeilkraut (Sagittaria), Sumpfried (Eleocharis), Simse (Scirpus), Paspalum, Ischaemum, Sphenoclea, Dactyloctenium, Straußgras (Agrostis), Fuchsschwanzgras (Alopecurus), Windhalm (Apera).

Monokotyle Kulturen der Gattungen: Reis (Oryza), Mais (Zea), Weizen (Triticum), Gerste (Hordeum), Hafer (Avena), Roggen (Secale), Mohrenhirse (Sorghum), Hirse (Panicum), Zuckerrohr (Saccharum), Ananas (Ananas), Spargel (Asparagus), Lauch (Allium).

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen z.B. Forst-, Ziergehölz-, Obst-, Wein-, Citrus-, Nuss-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Besonders geeignet sind die erfindungsgemäßen Wirkstoffe zur Bekämpfung von grasartigen Unkräutern vor dem Auflaufen der Pflanzen. Dabei ist ihre hohe Verträglichkeit für Kulturen wie Getreide, Mais, Rüben hervorzuheben.

Die erfindungsgemäßen Wirkstoffe können als solche oder in ihren Formulierungen auch in Mischung mit bekannten Herbiziden zur Unkrautbekämpfung Verwendung finden, wobei Fertigformulierung oder Tankmischung möglich ist.

Die Wirkstoffe können in die üblichen Formulierungen übergeführt werden, wie Lösungen, Emulsionen, Spritzpulver, Suspensionen, Pulver, Stäubemittel, Schäume, Pasten, lösliche Pulver, Granulate, Aerosole, Suspensions-Emulsionskonzentrate, Saatgutpuder, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä. sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe: natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate: gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehle, Kokosnußschalen, Maiskolben und Tabakstengel; als Emulgier- und/oder schaumerzeugende Mittel: nichtionogene und anionische Emulgatoren, wie Polyoxyäthylen-Fettsäure-Ester, Polyoxyäthylen-Fettalkohol-Äther, z.B. Alkylaryl-polyglykol-äther, Alkylsulfonate, Alkylsulfate, Arylsulfonate sowie Eiweißhydrolysate; als Dispergiermittel: z.B. Lignin—Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die aufgewandte Wirkstoffmenge kann in größeren Bereichen schwanken. Sie hängt im wesentlichen von der Art des gewünschten Effektes ab. Im allgemeinen liegen die Aufwandmengen zwischen 0,1 und 10 kg/Wirkstoff pro ha, vorzugsweise zwischen 0,2 und 5 kg/ha.

Beispiel A

Pre-emergence-Test

Lösungsmittel: 5 Gewichtsteile Aceton
Emulgator: 1 Gewichtsteil Alkylarylpolyglycoläther

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

Wirkstoffe, Aufwandmengen und Resultate gehen aus der nachfolgenden Tabelle hervor:

Tabelle A

Pre-emergence-Test

| Wirkstoff | Wirkstoff-aufwand kg/ha | Setaria | Lolium | Echino-chloa | Weizen | Hafer | Raps | Mais | Rüben |
|---|---|---|---|---|---|---|---|---|---|
| 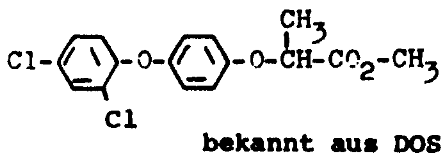 | 5 | 100 | 80 | 100 | 0 | 40 | 40 | 60 | 40 |
| | 2,5 | 90 | 60 | 90 | 0 | 20 | o | 40 | 20 |
| bekannt aus DOS 2 223 894 | | | | | | | | | |
| 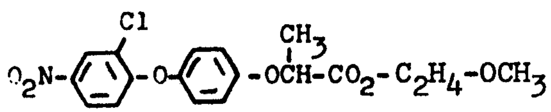 | 5 | 90 | 80 | 90 | 10 | 60 | 0 | 40 | 0 |
| | 2,5 | 90 | 70 | 90 | 0 | 40 | 0 | 20 | 0 |

Herstellungsbeispiele

Beispiel 1: 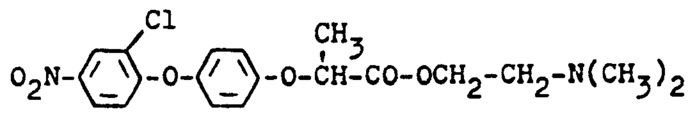

5 g (o,o6 Mol) 2-Dimethylamino-äthanol und lo ml Pyridin werden in 3o ml Toluol gelöst. Zu dieser Lösung tropft man bei 2o-3o$^{o}$C in ca. 3o Minuten eine Lösung von 17,8 g (o,o5 Mol) 4-(2-Chlor-4-nitro-phenoxy)-α-phenoxy-propionsäurechlorid in 3o ml Toluol. Dann wird die Reaktionslösung 8 Stunden bei 2o$^{o}$C gerührt. Zur Aufarbeitung versetzt man die Reaktionslösung mit 3oo ml Toluol, wäscht je einmal mit 2oo ml o,2 n Natronlauge und 2oo ml Wasser und destilliert dann das Lösungsmittel im Vakuum ab. Zurück bleiben 17,3 g (84 % der Theorie) eines orangefarbenen, viskosen Öls mit dem Brechungsindex $n_D^{20}$:1,569o an 4-(2-Chlor-4-nitro-phenoxy)-α-phenoxy-propionsäure-(2-dimethylamino-äthylester).

Beispiel 2: 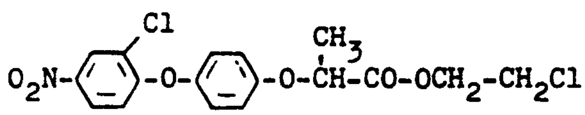

Zu einer Lösung von 17,8 g (o,o5 Mol) 4-(2-Chlor-4-nitro-phenoxy)-α-phenoxy-propionsäurechlorid, 5 ml Toluol und 4,8 g (o,o6 Mol) 2-Chloräthanol werden bei 0 - 5$^{o}$C in ca. 3o Minuten 15 ml Pyridin getropft. Anschließend wird 8 Stunden bei 2o$^{o}$C gerührt. Zur Aufarbeitung wäscht man die Reaktionslösung mit je 2oo ml o,2 n Natronlauge und 2oo ml Wasser und destilliert dann das Lösungsmittel im Vakuum ab. Zurück bleiben 16 g (8o % der Theorie) eines viskosen, orangefarbenen Öles mit dem Brechungsindex $n_D^{20}$: 1,5858 an 4-(2-Chlor-4-nitro-phenoxy)-α-phenoxy-propionsäure(2-chloräthylester).

Analog einem der vorstehend beschriebenen Verfahren können die folgenden Verbindungen hergestellt werden:

| Bei-spiel Nr. | Konstitution | Ausbeute (% der Theorie) | Physikal. Daten (Brechungsindex; Schmelzpunkt °C) |
|---|---|---|---|
| 3 | 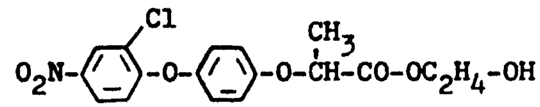 | 73,9 | $n_D^{20}$:1,5896 |
| 4 | 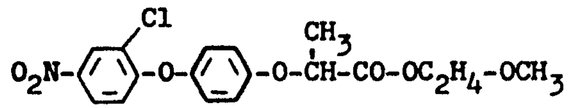 | 85 | $n_D^{20}$:1,5726 |
| 5 | 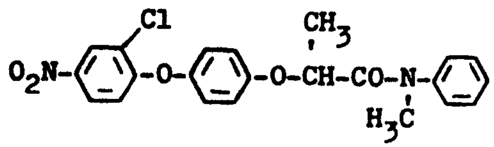 | 76 | 52-53 |

Beispiel 6: 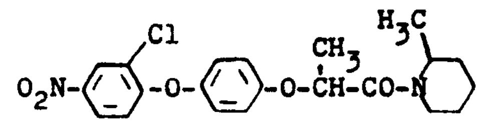

Zu einer Lösung von 17,8 g (o,o5 Mol) 4-(2-Chlor-4-nitro-phenoxy)-α-phenoxy-propionsäurechlorid in 5o ml Toluol werden bei 2o-3o°C in ca. 3o Minuten 15,6 g (o,1 Mol) 2-Methyl-piperidin zugetropft und anschließend 8 Stunden bei 2o°C nachgerührt. Dann werden 35o ml Toluol zugegeben, die organische Phase wird je einmal mit 2oo ml einer o,1 n Salzsäure und o,1 n Natronlauge gewaschen und dann wird das Lösungsmittel im Vakuum abdestilliert. Zurück bleiben 16 g (76 % der Theorie) eines viskosen Öles mit dem Brechungsindex $n_D^{2o}$: 1,5836.

Analog einer der Vorschriften der Beispiele 1 bis 6 können die folgenden Verbindungen hergestellt werden:

Beispiel Nr.

7 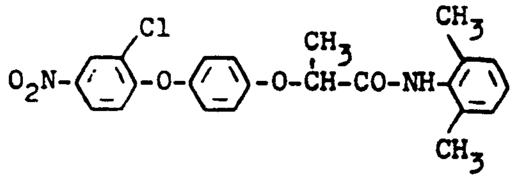

8 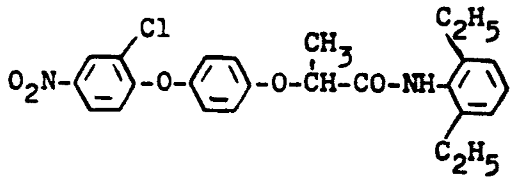

9 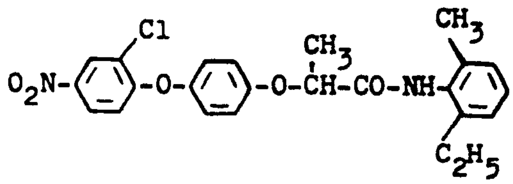

Bei-
spiel
Nr.

10 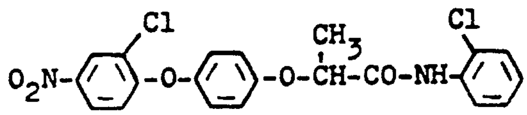

11 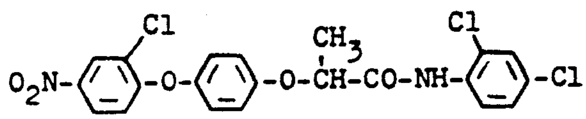

12 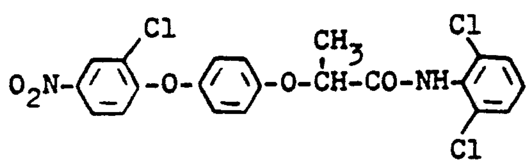

13 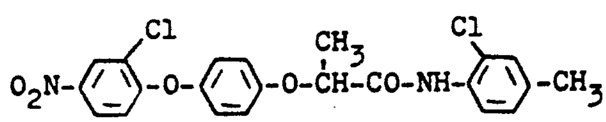

14 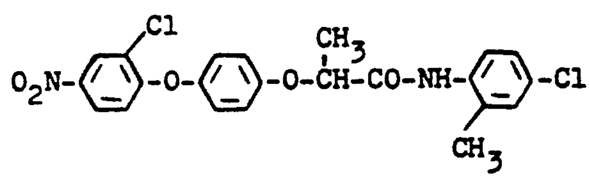

15 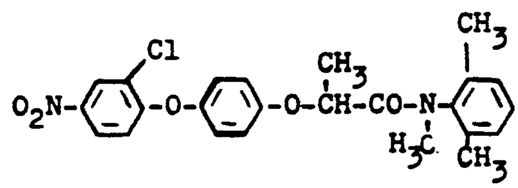

16 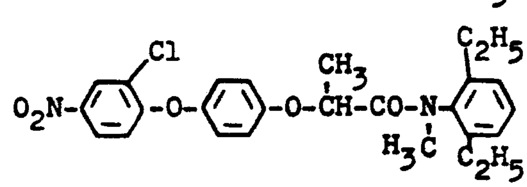

17 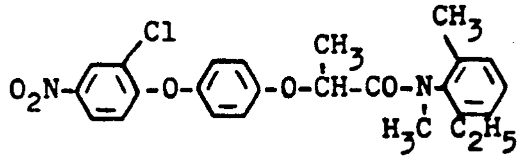

18 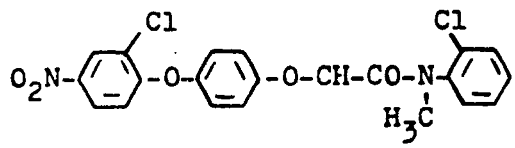

19 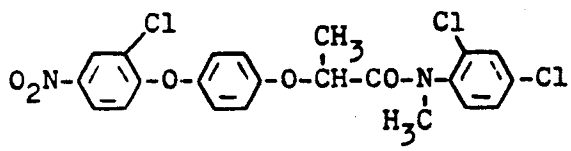

20 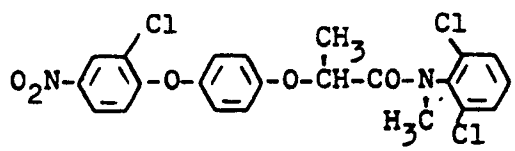

21 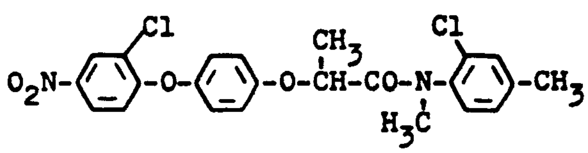

22 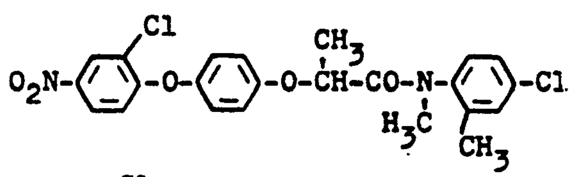

23 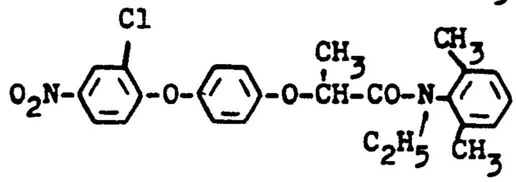

24 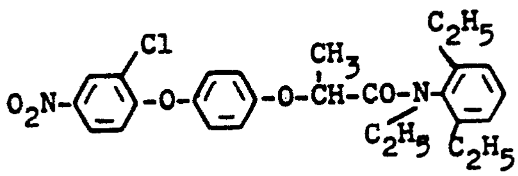

25 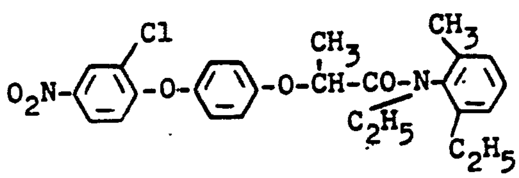

26 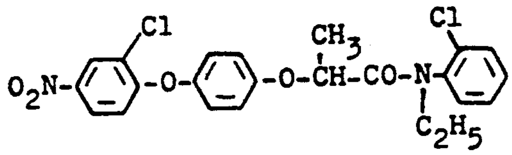

27 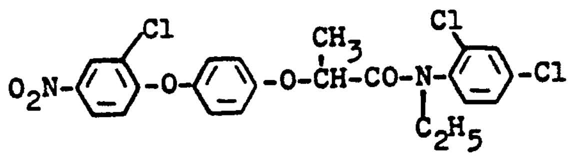

28 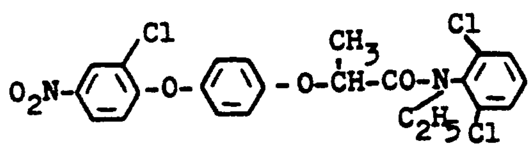

29 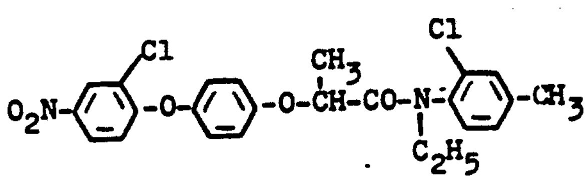

30 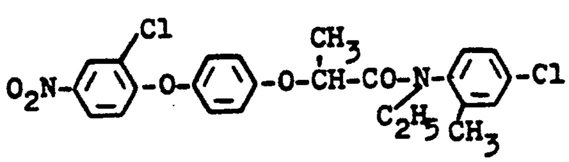

**Beispiel 31:** 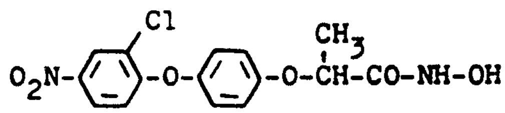

Zu 34 g 4-(2-Chlor-4-nitrophenoxy)-α-phenoxypropionsäure-methylester (o,o97 Mol) und 1oo ml Methanol werden bei 1o°C 8,9 g Hydroxylamin-Hydrochlorid und 15 ml Wasser zugetropft. Anschließend gibt man bei einer Temperatur von o-1o°C 9,2 g Natriumhydroxyd in 12 ml Wasser zu. Dann wird in einen Liter Wasser gegossen, mit Eisessig auf pH 5 gestellt und anschließend das Kristallisat abgesaugt. Man erhält so 3o,2 g (88,3% der Theorie) eines blaßgelben Produktes mit dem Schmelzpunkt 127°C.

Herstellung des als Ausgangsprodukt benötigten 4-(2-Chlor-4-nitrophenoxy)-α-phenoxypropionsäure-methylesters:

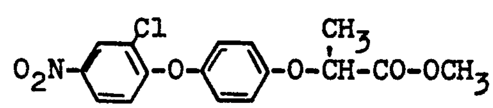

Eine Mischung aus 398,3 g (1,5 Mol) 2-Chlor-4-nitro-4'-hydroxy-diphenyläther, 240 g Kaliumcarbonat und 1,5 l Dimethylsulfoxid wird bei 90 - 100°C mit 211,3 g Chlorpropionsäuremethylester versetzt und 12 Stunden bei 90 - 100°C nachgerührt. Danach gießt man die Reaktionsmischung auf 16 l Wasser und saugt ab. Der Rückstand wird aus Methanol umkristallisiert und man erhält 190 g ( 35 % der Theorie) beiger Kristalle mit dem Schmelzpunkt 71°C.

Nach einem oder mehreren der vorstehend beschriebenen Verfahren können auch die in den folgenden Beispielen formelmäßig aufgeführten Verbindungen hergestellt werden:

| Beispiel-Nr. | Strukturformel | Brechungsindex bzw. Schmelzp. | Ausbeute in % |
|---|---|---|---|
| 32 | 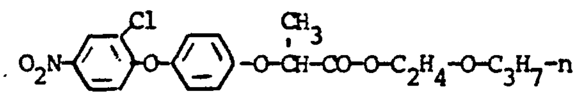 | 1,5585 | 74 |
| 33 | 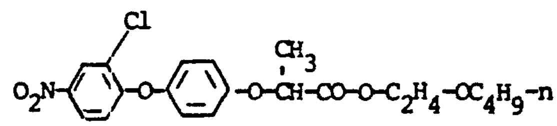 | 1,556 | 71 |
| 34 | 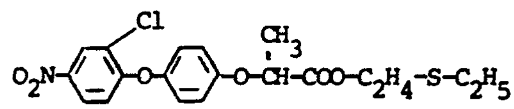 | 1,5845 | 72,7 |
| 35 | 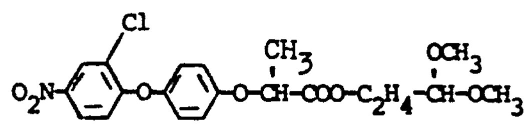 | 1,5595 | 70,1 |
| 36 | 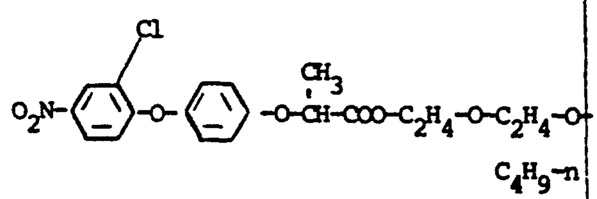 | 1,5478 | 77,6 |
| 37 | 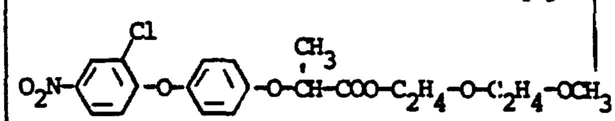 | 1,56 | |

| Beispiel-Nr. | Strukturformel | Brechungsindex bzw. Schmelzp. | Ausbeute in % |
|---|---|---|---|
| 38 | 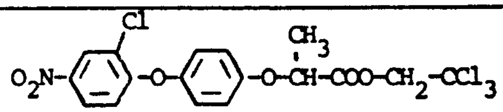 | 78°C | 66,8 |
| 39 | 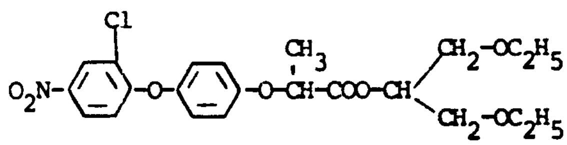 | 1,5465 | 54,5 |
| 40 | 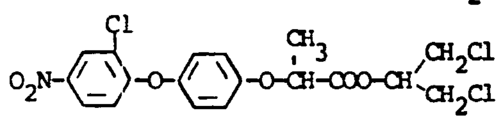 | 1,5822 | 71,3 |
| 41 | 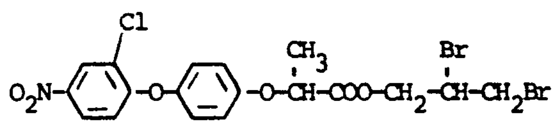 | 1,5994 | 51,4 |
| 42 | 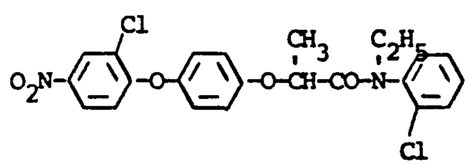 | 1,601 | 68,2 |
| 43 | 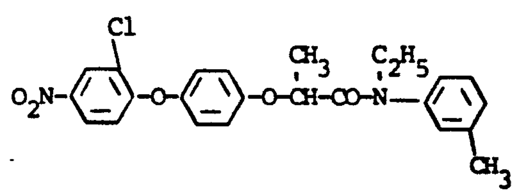 | 91° | 65,4 |
| 44 | 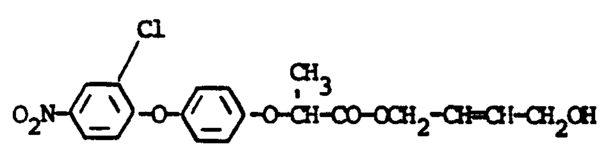 | 1,584 | 68,7 |
| 45 | 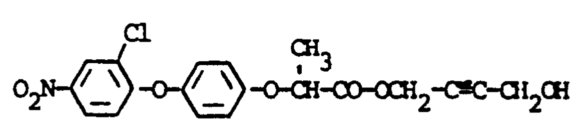 | 96°C | 36,2 |

| Beispiel-Nr. | Strukturformel | Brechungsindex bzw. Schmelzpunkt | Ausbeute in % |
|---|---|---|---|
| 46 | 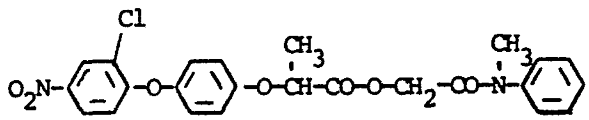 | 1,5845 | 65,2 |

Die als Ausgangsprodukte benötigten 4-(2-Chlor-4-nitro-phenoxy)-phenoxyalkancarbonsäurehalogenide (II) können wie im folgenden beschrieben hergestellt werden:

a) 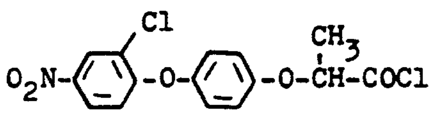

267 g (o,79 Mol) 4-(2-Chlor-4-nitro-phenoxy)-α-phenoxy-propionsäure und 5 ml Dimethylformamid als Katalysator werden in 8oo ml Äthylenchlorid gelöst. Bei 2o-3o°C werden dann 119 g Thionylchlorid in ca. 2 Stunden hinzugetropft. Dann wird die Reaktionslösung langsam zum Rückfluß erhitzt. Zur Aufarbeitung wird die Reaktionslösung filtriert, überschüssiges Thionylchlorid wird abdestilliert, der ölige Rückstand wird mit 1 l Diäthyläther extrahiert und der Äther danach abdestilliert. Zurück bleiben 251 g (91 % der Theorie) an 4-(2-Chlor-4-nitro-phenoxy)-α-phenoxy-propionsäurechlorid als dunkles Öl, das nach längerem Stehen kristallisiert. Der Schmelzpunkt ist 58°C.

Die weiterhin zu verwendenden 2-Chlor-4-nitro-4'-hydroxy-diphenyläther (IV) werden folgendermaßen hergestellt:

a[1])

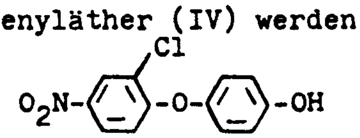

lo,5 kg Hydrochinon (95,5 Mol), 3,5 kg Calziumhydroxyd, 2oo g Natriumsulfit und 5o kg Dimethylsulfoxyd werden in einem Rührkessel (RKI) unter Stickstoffatmosphäre auf 8o-85°C erhitzt. Während 6 Stunden werden dann bei 8o-85°C 9,15 kg 3,4-Dichlornitrobenzol, gelöst in 15 l Dimethylsulfoxyd, zugegeben. Anschließend wird noch 3 Stunden bei 8o°C nachgerührt. In einem zweiten Rührkessel (RK II) werden 4oo ml Wasser und 15 kg konzentrierter Salzsäure vorgelegt. Unter Rühren läßt man dann innerhalb 1 Stunde die Reaktionslösung aus dem RK I in den RK II einlaufen. Das Reaktionsprodukt fällt kristallin aus und wird mit 5o l Wasser neutral gewaschen. Es werden 12,5 kg (99 % der Theorie) erhalten an 2-Chlor-4-nitro-4'-hydroxy-diphenyläther mit dem Schmelzpunkt 151°C.

b[1])

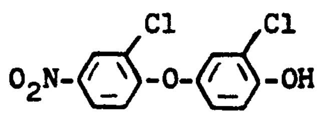

Zu einer Mischung aus 4oo g (1,5 Mol) der unter a[1]) hergestellten Verbindung und 15 g Eisen (III)chlorid gibt man 5oo ml Sulfonylchlorid und läßt 45 Stunden bei Raumtemperatur rühren. Danach gibt man langsam unter Kühlen 7oo ml Wasser zu, wobei die Temperatur auf 35°C ansteigt. Danach gibt man nochmals 3 l Wasser zu und saugt das sich ausscheidende Produkt ab. Man erhält 443 g (98,4 % der Theorie) 2-Chlor-4-nitro-3'-chlor-4'-hydroxy-diphenyläther mit einem Schmelzpunkt von lo8°C.

## Patentansprüche

1. 4-(2-Chlor-4-nitrophenoxy)-phenoxy-alkancarbonsäure-Derivate der Formel I

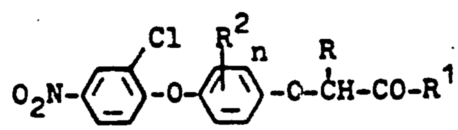

(I)

in welcher

R für Wasserstoff oder Alkyl steht,

$R^1$ für Akoxy, das ein- oder mehrfach durch Hydroxyl, Halogen, Akoxy, Alkoxy-alkoxy, Akylthio, Amino, Monoalkylamino, Dialkylamino oder Carbonyl-N-alkyl-N-aryl substituiert ist, für durch Hydroxy-alkenoxy, durch Hydroxy-alkinoxy, Hydroxylamino, einen stickstoffhaltigen Heterocyclus, dessen Ring durch weitere Heteroatome unterbrochen sein kann und/oder gegebenenfalls durch Alkyl oder Halogen substituiert sein kann, Arylamino bzw. Monoalkyl-monoarylamino, in denen der Arylrest jeweils durch Alkyl, Nitro, Halogen oder Halogenalkyl substituiert sein kann steht,

$R^2$ für Wasserstoff, Halogen oder Alkyl steht und

n für eine ganze Zahl von 1 bis 4 steht.

2. Verfahren zur Herstellung der 4-(2-Chlor-4-nitrophenoxy)-phenoxyalkancarbonsäure-Derivate der Formel gemäß Anspruch 1, dadurch gekennzeichnet, daß man

a) 4-(2-Chlor-4-nitrophenoxy)-phenoxyalkancarbonsäurehalogenide der Formel II

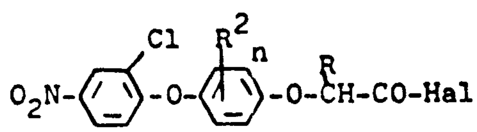

(II)

in welcher

$R$, $R^2$ und $n$ die oben angegebene Bedeutung haben und
Hal für Halogen, vorzugsweise Chlor, steht,

mit Verbindungen der Formel III

$HR^1$ (III)

in welcher

$R^1$ die oben angegebene Bedeutung hat,

gegebenenfalls in Gegenwart eines Säureakzeptors oder gegebenenfalls in Form der Alkali-, Erdalkali- oder Ammoniumsalze und gegebenenfalls in Gegenwart eines Lösungs- oder Verdünnungsmittels umsetzt, oder

b) 2-Chlor-4-nitro-4'-hydroxy-diphenyläther der Formel IV

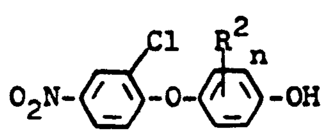

(IV)

in welcher

$R^2$ und n die oben angegebene Bedeutung haben, gegebenenfalls in Form der Alkali- bzw. Erdalkalisalze

mit α-Halogenalkancarbonsäuren oder Derivaten der Formel V

$$Hal^1\text{-}\overset{R}{\overset{|}{C}}H\text{-}CO\text{-}R^1 \qquad (V)$$

in welcher

R und $R^1$ die oben angegebene Bedeutung haben und

$Hal^1$ für Halogen steht,

gegebenenfalls in Gegenwart eines Säureakzeptors und gegebenenfalls in Gegenwart eines Lösungs- oder Verdünnungsmittels umsetzt oder

c) 4-(2-Chlor-4-nitrophenoxy)-phenoxyalkancarbonsäuremethylester der FormelVI

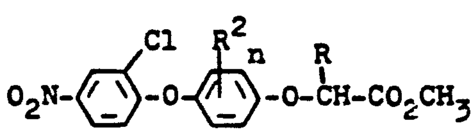

(VI)

in welcher .

R, $R^2$ und n die oben angegebene Bedeutung haben,

mit Alkoholen der Formel VII

$$HOR^3 \qquad (VII)$$

in welcher

$R^3$ für substituiertes Alkyl mit mehr als einem Kohlenstoffatom steht,

gegebenenfalls in Gegenwart eines Alkoholats und gegebenenfalls in Gegenwart eines Lösungs- oder Verdünnungsmittels umsetzt, oder

d) die Verbindungen der Formel (VI) mit Hydroxylamin-Hydrohalogenid der Formel VIII

$$H_2NOH \times HHalogen \qquad (VIII)$$

gegebenenfalls in Gegenwart eines Säureakzeptors und gegebenenfalls in Gegenwart eines Lösungs- oder Verdünnungsmittels umsetzt.

3. Herbizide Mittel, gekennzeichnet durch einen Gehalt an 4-(2-Chlor-4-nitrophenoxy)-phenoxy-alkancarbonsäure-Derivaten gemäß Anspruch 1.

4. Verwendung von 4-(2-Chlor-4-nitrophenoxy)-phenoxy-alkancarbonsäure-Derivaten gemäß Anspruch 1 zur Bekämpfung von Unkräutern.

5. Verfahren zur Bekämpfung von Unkräutern, dadurch gekennzeichnet, daß man 4-(2-Chlor-4-nitrophenoxy)-phenoxy-alkancarbonsäure-Derivate gemäß Anspruch 1 auf Unkräuter und/oder ihren Lebensraum einwirken läßt.

6. Verfahren zur Herstellung herbizider Mittel, dadurch gekennzeichnet, daß man 4-(2-Chlor-4-nitrophenoxy)-phenoxy-alkancarbonsäure-Derivate gemäß Anspruch 1 mit Streckmitteln und/oder oberflächenaktiven Stoffen vermischt.

Europäisches Patentamt

# EUROPÄISCHER RECHERCHENBERICHT

0000050

Nummer der Ar
EP 78 1(

## EINSCHLÄGIGE DOKUMENTE

| ıtegorie | Kennzeichnung des Dokuments mit Angabe, soweit erforderlich, der maßgeblichen Teile | betrifft Anspruch |
|---|---|---|
| D | DE - A - 2 652 384 (CIBA-GEIGY)<br>* Seite 6; Seite 7; Seite 15, Beispiel 1; Seite 10; letzter Abschnitt; Seite 11; Abschnitte 1,2; Seite 18, Seite 4, Ansprüche 14,16 *<br>-- | 1,2,3-6 |
| | FR - A - 2 325 638 (ISHIHARA SANGYO KAISHA)<br>* Seite 1, Zeilen 1-28; Seite 3, Seite 4, Zeilen 16-29; Seite 16, Zeilen 25-40; Seite 17; Seite 18, Zeilen 1-10 *<br>---- | 1,2,3-6 |

KLASSIFIKATION DER ANMELDUNG (Int.Cl.²)

C 07 C 79/46
A 01 N 9/26
A 01 N 9/12
A 01 N 9/22
A 01 N 9/20
C 07 C 103/178
C 07 C 103/34

RECHERCHIERTE SACHGEBIETE (Int. Cl.²)

C 07 C 69/67
C 07 C 79/46
C 07 C 59/25

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

Der vorliegende Recherchenbericht wurde für alle Patentansprüche erstellt.

| cherchenort | Abschlußdatum der Recherche | Prüfer |
|---|---|---|
| Den Haag | 31-08-1978 | KINZINGER |